# EUROPEAN PATENT APPLICATION

(11) **EP 2 433 570 A1**
(43) Date of publication of application: **28.03.2012**
(21) Application number: 11163081.0
(22) Date of filing: 19.04.2011
(51) Int. Cl.: A61B 8/08, G06T 7/00

(54) **Ultrasound system for displaying slice of object and method thereof**

(30) Priority: 28.09.2010 KR 20100093682
(71) Applicant: Samsung Medison Co., Ltd., Nam-myun Hongchun-gun Kangwon do 250-875 (KR)
(72) Inventor: Kim, Sung Yoon, 472-735, Gyeonggi-Do (KR); Ahn, Mi Jeong, 120-160, Seoul (KR)
(74) Representative: Schmid, Wolfgang

(57) **Abstract**

Provided are an ultrasound system for displaying a slice of an object and a method thereof, which may extract a plurality of slice images from a volume image obtained by scanning the nuchal translucency of a fetus, may determine, as an effective slice image, a slice image having a meaningful measurement value among values measured in each of the extracted slice images, and may identify a risk of Down syndrome of the fetus using the measurement value in the effective slice image.

## Description

### CROSS-REFERENCE TO RELATED APPLICATION

This application claims the benefit of Korean Patent Application No. 10-2010-0093682, filed on September 28, 2010, in the Korean Intellectual Property Office, the disclosure of which is incorporated herein by reference.

### BACKGROUND

### 1. Field of the Invention

The present invention relates to a technique for easily determining an effective measurement value of a predetermined measurement region in a volume image of an object scanned by an ultrasound system.

### 2. Description of the Related Art

An ultrasound system is an apparatus for transmitting an ultrasound wave signal toward a predetermined structure (that is, an object such as a fetus or an internal organ) inside the body from the surface of the body and for visualizing a cross section of soft tissues or a blood flow using information of the ultrasound wave signal reflected from the tissues of the body.

This ultrasound system has advantages of a small size, a low cost, a real-time display, and a high stability without exposing a subject or a user to X-ray radiation, and thus, the ultrasound system is widely used along with other diagnostic imaging systems such as an X-ray diagnosis equipment, a computerized tomography (CT) scanner, a magnetic resonance imaging (MRI) equipment, a nuclear medicine diagnosis equipment, and the like.

A general method for determining Down syndrome in a fetus is to measure a thickness of the nuchal translucency (NT) of the fetus using an ultrasound system. The method was developed by Nicolaides in 1992, and uses a known phenomenon that when a fetus has an abnormality, fluid is accumulated under skin around the neck of the fetus and the NT is thick.

In particular, when a fetus has a risk of chromosomal abnormalities including Down syndrome or anomalies of the heart, the NT of the fetus is thick in many cases. Accordingly, a doctor measures a thickness of a transparent space behind the neck of the fetus using an ultrasound system, and when the nuchal thickness is larger than 2.5 mm, the doctor will diagnose abnormalities of the fetus using an in-depth test such as chorionic villus sampling or amniocentesis.

In some instances, however the thickness of the NT may not be measured accurately depending on an angle or a measuring method. Accordingly, there is a desire for a solution to easily diagnose abnormalities of a fetus through accurate measurement of the NT thickness of the fetus.

### SUMMARY

An aspect of the present invention provides an ultrasound system for displaying a slice of an object and a method thereof, which may extract a plurality of slice images from a volume image obtained by scanning the nuchal translucency (NT) of a fetus, and may measure a thickness of the NT using, as an effective slice image, a slice image having a meaningful measurement value among values measured in each of the extracted slice images, thereby providing a more accurate measurement of the NT thickness of the fetus.

Another aspect of the present invention provides an ultrasound system for displaying a slice of a 3-dimensional (3D) object and a method thereof, which may obtain unit images of a measurement region from 'n' slice images, and may perform a 3D modeling process by applying the obtained unit images to a 3D coordinate system, thereby displaying 3D slice images of the measurement region.

According to an aspect of the present invention, there is provided an ultrasound system for displaying a slice of an object including an image scanning unit to generate a volume image through scanning of an object, a measuring unit to extract 'n' slice images of a measurement region in the volume image, and to determine a measurement value associated with the measurement region in each of the slice images, 'n' being a natural number, and a processor to determine an effective slice image among the 'n' slice images using the measurement value.

In this instance, the measuring unit may obtain a measurement value by measuring the measurement region at each of 'm' measuring points in one slice image, 'm' being a natural number, and may determine a relatively smaller value among the obtained 'm' measurement values.

The processor may determine, as an effective slice image, a slice image having a relatively larger measurement value among the 'm' measurement values determined in each of the slice images.

The ultrasound system may further include a modeling unit to obtain unit images of the measurement region from the 'n' slice images and to perform a 3D modeling process by applying the obtained unit images to a 3D coordinate system.

When the object is the fetus and the measurement region is the NT, the measuring unit may set a seed around the NT of the fetus in the volume image, and may extract the slice images by cutting the NT into 'n' slices based on the set seed.

In this instance, the measuring unit may determine 'n' measurement values of the NT thickness in each of the slice images. The processor may determine, as the effective slice image, a slice image having a relatively larger measurement value among the 'n' measurement values.

According to another aspect of the present invention, there is provided a method for displaying a slice of an object in an ultrasound system including generating a volume image through scanning of an object, extracting 'n' slice images of a measurement region in the volume image, 'n' being a natural number, determining a measurement value associated with the measurement region in each of the slice images, and determining an effective slice image among the 'n' slice images using the measurement value.

### EFFECT OF THE INVENTION

According to an aspect of the present invention, provided is an ultrasound system and a method thereof, which may extract a plurality of slice images from a volume image obtained by scanning the nuchal translucency (NT) of a fetus, and may measure a thickness of the NT using, as an effective slice image, a slice image having a meaningful measurement value among values measured in each of the extracted slice images, thereby providing a more accurate measurement of the NT thickness of the fetus.

According to another aspect of the present invention, provided is an ultrasound system and a method thereof, which may obtain unit images of a measurement region from 'n' slice images, and may perform a 3-dimensional (3D) modeling process by applying the obtained unit images to a 3D coordinate system, thereby displaying 3D slice images of the measurement region.

### BRIEF DESCRIPTION OF THE DRAWINGS

These and/or other aspects, features, and advantages of the invention will become apparent and more readily appreciated from the following description of exemplary embodiments, taken in conjunction with the accompanying drawings of which:
FIG. 1 is a block diagram illustrating an internal structure of an ultrasound system for displaying a slice of an object according to an embodiment of the present invention;
FIG. 2 is a view illustrating an example of extraction of a slice image from a volume image of an object; and
FIG. 3 is a flowchart illustrating a method for displaying a slice of an object in the ultrasound system of FIG. 1.

### DETAILED DESCRIPTION

Reference will now be made in detail to exemplary embodiments of the present invention, examples of which are illustrated in the accompanying drawings, wherein like reference numerals refer to the like elements throughout. Exemplary embodiments are described below to explain the present invention by referring to the figures.

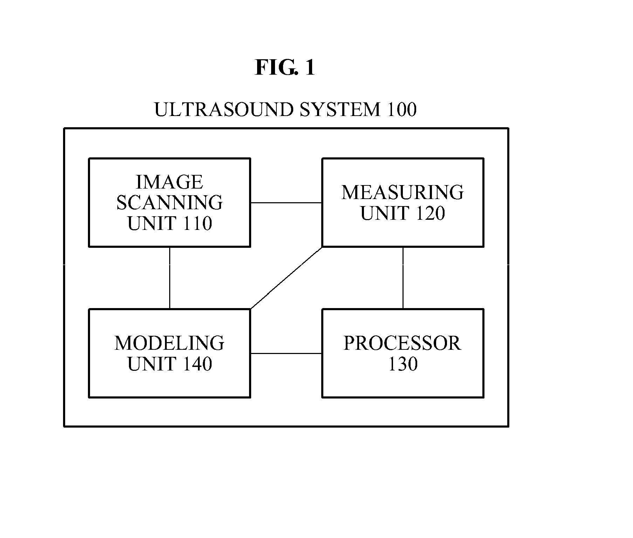

FIG. 1 is a block diagram illustrating an internal structure of an ultrasound system 100 for displaying a slice of an object according to an embodiment of the present invention.

Referring to FIG. 1, the ultrasound system 100 for displaying a slice of an object (hereinafter referred to as 'ultrasound system') according to an embodiment of the present invention may include an image scanning unit 110, a measuring unit 120, a processor 130, and a modeling unit 140.

The image scanning unit 110 may generate a volume image through scanning of an object. For example, the image scanning unit 110 may generate a volume image that provides a 3-dimensional (3D) representation of image data obtained by scanning an object in the body. In this instance, the object in the body may include a fetus, an internal organ, and the like. That is, the image scanning unit 110 may generate a volume image through scanning of a fetus, an internal organ, and the like.

As an example of generation of a volume image, the image scanning unit 110 may set a region of interest (ROI) in an object and may locate a seed within the set ROI. In this instance, when the object is a fetus, the seed may be located near the nuchal translucency (NT) of the fetus. Subsequently, the image scanning unit 110 may generate a volume image of the object by creating image data through scanning of the object using a 3D ultrasound wave and by representing the created image data in three dimensions.

The measuring unit 120 may extract 'n' slices images of a measurement region in the volume image, 'n' being a natural number, and may determine a measurement value associated with the measurement region in each of the slice images. Here, the measurement region may be the NT of a fetus. That is, when the object is a fetus and the measurement region is the NT, the measuring unit 120 may set a seed near the NT of the fetus in the volume image, and may extract slice images by cutting the NT into 'n' slices based on the set seed.

According to an embodiment, the measuring unit 120 may extract a plurality of slice images, that is, 'n' slice images of the measurement region, that is, the NT. The measuring unit 120 may obtain a measurement value by measuring the measurement region at each of 'm' measuring points in one slice image, 'm' being a natural number. For example, the measurement value may be a measured thickness of the NT. The measuring unit 120 may determine a relatively smaller value among the obtained 'm' measurement values.

For example, when the measuring unit 120 obtains a measurement value at each of at least two measuring points in a first slice image among the 'n' slice images, the measuring unit 120 may determine, as a measurement value of the first slice image, a relatively smaller value among the at least two measurement values.

As described above, measuring of the NT thickness of a fetus is generally used for identifying a risk of Down syndrome in the fetus. When a fetus has a risk of chromosomal abnormalities including Down syndrome or anomalies of the heart, the NT of the fetus is thick in many cases. Accordingly, a doctor measures a thickness of a transparent space behind the neck of the fetus using the ultrasound system 100, and when the NT thickness is larger than 2.5 mm, the doctor will diagnose abnormalities of the fetus using an in-depth test such as chorionic villus sampling or amniocentesis.

However, the thickness of the NT for determining a risk of Down syndrome may vary depending on a location of the fetus and an anatomical part of the fetus to be scanned by the ultrasound system 100. Thus, it may be difficult to accurately measure the thickness of the NT. Accordingly, to provide an accurate measurement of the thickness of the NT, the thickness of the NT may be measured after cutting the NT into 'n' slices, thereby accurately identifying a risk of abnormalities of the fetus.

The processor 130 may determine, as an 'effective slice image', a slice image having a relatively larger measurement value among 'n' measurement values determined in each of the slice images. The effective slice image may be an image of which a measurement value has a meaning, among the plurality of slice images. That is, a measurement value measured in an effective slice image may reflect an accurate measurement of the NT thickness, among measurement values measured in the plurality of slice images.

According to an embodiment, when the measuring unit 120 determines 'n' measurement values of the NT thickness in each of the slice images, the processor 130 may determine, as an effective slice image, a slice image having a relatively larger measurement value among the 'n' measurement values. For example, when a measurement value measured in a first slice image is 2.0 mm, a measurement value measured in a second slice image is 2.2 mm, and a measurement value measured in a third slice image is 2.3 mm, the processor 130 may determine, as an effective slice image, the third slice image among the first slice image to the third slice image.

Accordingly, the ultrasound system 100 may accurately measure the NT thickness by identifying a measurement value measured in the determined effective slice image as the NT thickness of the fetus.

According to another embodiment, the modeling unit 140 may obtain unit images of the measurement region from the 'n' slice images, and may perform a 3D modeling process by applying the obtained unit images to a 3D coordinate system. That is, the modeling unit 140 may generate 3D slice images of the NT of the fetus by applying 2D unit images of the NT to a 3D coordinate system. The generated 3D slice images may be displayed on a screen of the ultrasound system 100.

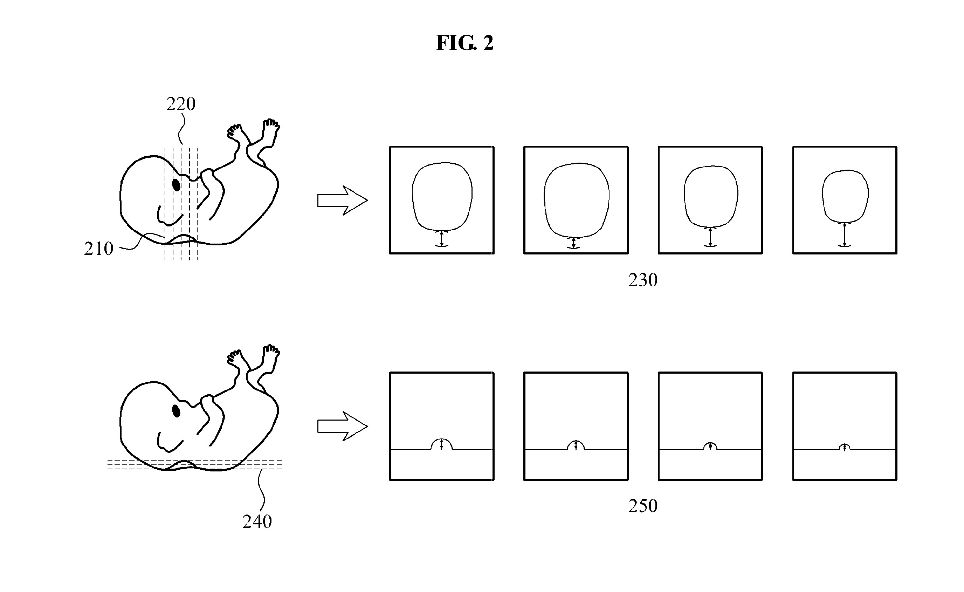

FIG. 2 is a view illustrating an example of extraction of a slice image from a volume image of an object.

Referring to FIG. 2, the measuring unit 120 may extract, as a slice image 230, a 'longitudinal' section 220 of a measurement region, that is, the NT 210 in a volume image of an object. The measuring unit 120 may obtain a measurement value by measuring a measurement region at each of 'm' measuring points in each of the extracted slice images 230, and may determine, as a measurement value representing the slice image 230, a relatively smaller value among the obtained 'm' measurement values, 'm' being a natural number.

According to another embodiment, the measuring unit 120 may extract, as a slice image 250, a 'latitudinal' section 240 of a measurement region, that is, the NT 210 in a volume image of an object. The measuring unit 120 may obtain a measurement value by measuring a measurement region at each of 'm' measuring points in each of the extracted slice images 250, and may determine, as a measurement value representing the slice image 250, a relatively smaller value among the obtained 'm' measurement values, 'm' being a natural number.

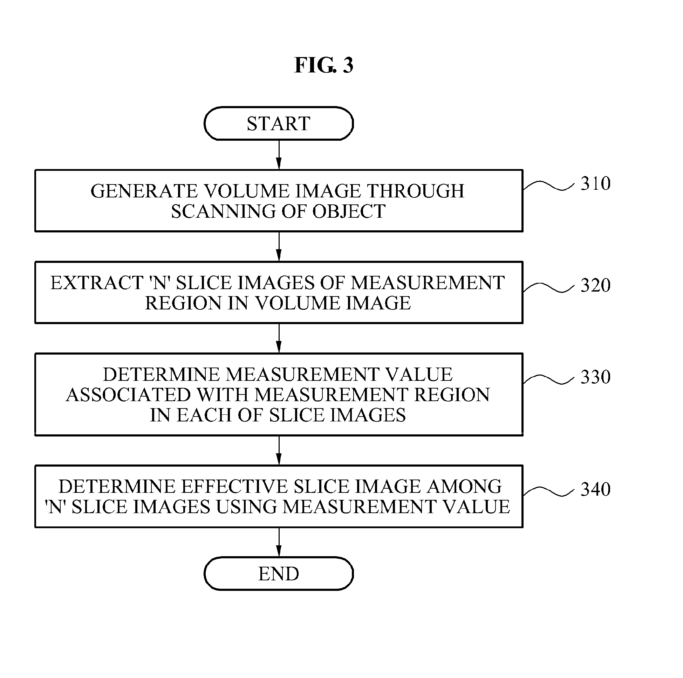

FIG. 3 is a flowchart illustrating a method for displaying a slice of an object in the ultrasound system 100 of FIG. 1.

Referring to FIG. 3, the ultrasound system 100 may generate a volume image through scanning of an object, in operation 310. The ultrasound system 100 may generate a volume image that provides a 3D representation of image data obtained by scanning an object in the body. Here, the object in the body may include a fetus, an internal organ, and the like. That is, the ultrasound system 100 may generate a volume image through scanning of a fetus, an internal organ, and the like.

In operation 320, the ultrasound system 100 may extract 'n' slice images of a measurement region in the volume image, 'n' being a natural number. Referring to FIG. 2, the ultrasound system 100 may extract, as a slice image, a 'longitudinal' or 'latitudinal' section of a measurement region, that is, the NT in the volume image of the object.

In operation 330, the ultrasound system 100 may determine a measurement value associated with the measurement region in each of the slice images. The ultrasound system 100 may obtain a measurement value by measuring the measurement region at each of 'm' measuring points in one slice image, 'm' being a natural number. For example, the measurement value may be a measured thickness of the NT. The ultrasound system 100 may determine, as a measurement value representing the slice image, a relatively smaller value among the obtained 'm' measurement values.

In operation 340, the ultrasound system 100 may determine an effective slice image among the 'n' slice images using the measurement value. The ultrasound system 100 may determine, as an effective slice image, a slice image having a relatively larger measurement value among 'n' measurement values determined in each of the slice images.

Accordingly, the ultrasound system 100 may accurately measure the thickness of the NT by identifying the measurement value measured in the determined effective slice image as the NT thickness of the fetus.

According to another embodiment, the ultrasound system 100 may obtain unit images of the measurement region from the 'n' slice images, may perform a 3D modeling process by applying the obtained unit images to a 3D coordinate system to generate 3D slice images of the measurement region, and may display the generated 3D slice images on a screen.

The above-described exemplary embodiments of the present invention may be recorded in non-transitory computer-readable media including program instructions to implement various operations embodied by a computer. The media may also include, alone or in combination with the program instructions, data files, data structures, and the like. Examples of non-transitory computer-readable media include magnetic media such as hard disks, floppy disks, and magnetic tape; optical media such as CD ROM disks and DVDs; magneto-optical media such as optical disks; and hardware devices that are specially configured to store and perform program instructions, such as read-only memory (ROM), random access memory (RAM), flash memory, and the like. Examples of program instructions include both machine code, such as produced by a compiler, and files containing higher level code that may be executed by the computer using an interpreter. The described hardware devices may be configured to act as one or more software modules in order to perform the operations of the above-described exemplary embodiments of the present invention, or vice versa.

Although a few exemplary embodiments of the present invention have been shown and described, the present invention is not limited to the described exemplary embodiments. Instead, it would be appreciated by those skilled in the art that changes may be made to these exemplary embodiments without departing from the principles and spirit of the invention, the scope of which is defined by the claims and their equivalents.

## Claims

1. An ultrasound system for displaying a slice of an object, the ultrasound system comprising:
an image scanning unit to generate a volume image through scanning of an object;
a measuring unit to extract 'n' slice images of a measurement region in the volume image, and to determine a measurement value associated with the measurement region in each of the slice images, 'n' being a natural number; and
a processor to determine an effective slice image among the 'n' slice images using the measurement value.

2. The ultrasound system of claim 1, wherein the measuring unit obtains a measurement value by measuring the measurement region at each of 'm' measuring points in one slice image, 'm' being a natural number, and determines a relatively smaller value among the obtained 'm' measurement values.

3. The ultrasound system of claim 1, wherein the processor determines, as an effective slice image, a slice image having a relatively larger measurement value among the 'm' measurement values determined in each of the slice images.

4. The ultrasound system of claim 1, further comprising:
a modeling unit to obtain unit images of the measurement region from the 'n' slice images and to perform a 3-dimensional (3D) modeling process by applying the obtained unit images to a 3D coordinate system.

5. The ultrasound system of claim 1, wherein when the object is the fetus and the measurement region is the nuchal translucency (NT), the measuring unit sets a seed around the NT of the fetus in the volume image, and extracts the slice images by cutting the NT into 'n' slices based on the set seed.

6. The ultrasound system of claim 5, wherein the measuring unit determines 'n' measurement values of the NT thickness in each of the slice images, and
the processor determines, as the effective slice image, a slice image having a relatively larger measurement value among the 'n' measurement values.

7. A method for displaying a slice of an object in an ultrasound system, the method comprising:
generating a volume image through scanning of an object;
extracting 'n' slice images of a measurement region in the volume image, 'n' being a natural number;
determining a measurement value associated with the measurement region in each of the slice images; and
determining an effective slice image among the 'n' slice images using the measurement value.

8. The method of claim 7, wherein the determining of the measurement value associated with the measurement region comprises:
obtaining a measurement value by measuring the measurement region at each of 'm' measuring points in one slice image, 'm' being a natural number; and
determining a relatively smaller value among the obtained 'm' measurement values.

9. The method of claim 7, wherein the determining of the effective slice image comprises determining, as the effective slice image, a slice image having a relatively larger measurement value among the 'n' measurement values determined in each of the slice images.

10. The method of claim 7, further comprising:
obtaining unit images of the measurement region from the 'n' slice images; and
performing a 3D modeling process by applying the obtained unit images to a 3D coordinate system.

11. The method of claim 7, wherein when the object is the fetus and the measurement region is the NT, the extracting of the slice images comprises:
setting a seed around the NT of the fetus in the volume image; and
extracting the slice images by cutting the NT into 'n' slices based on the set seed.

12. The method of claim 11, wherein the determining of the measurement value associated with the measurement region comprises determining 'n' measurement values of the NT thickness in each of the slice images, and
the determining of the effective slice image comprises determining, as the effective slice image, a slice image having a relatively larger measurement value among the 'n' measurement values.
